# Europäisches Patentamt
# European Patent Office
# Office européen des brevets

(19)

(11) Veröffentlichungsnummer: **0 000 894**
**B1**

(12)

# EUROPÄISCHE PATENTSCHRIFT

(45) Veröffentlichungstag der Patentschrift: **15.07.81**

(51) Int. Cl.³: **C 07 C 68/08, C 07 C 69/96**

(21) Anmeldenummer: **78100607.7**

(22) Anmeldetag: **07.08.78**

(54) Verfahren zur Abtrennung von Dimethylcarbonat aus seinem Azeotrop mit Methanol.

(30) Priorität: **18.08.77 DE 2737265**

(43) Veröffentlichungstag der Anmeldung:
**07.03.79 Patentblatt 79/5**

(45) Bekanntmachung des Hinweises auf die Patenterteilung:
**15.07.81 Patentblatt 81/28**

(84) Benannte Vertragsstaaten:
**CH DE FR GB NL**

(56) Entgegenhaltungen:
**DE - A - 2 528 412**
**DE - B - 1 094 724**
**FR - A - 1 490 688**
**US - A - 3 239 435**
**US - A - 3 940 450**

**L. H. HORSLEY: "Azeotropic Data-III"**
**1973, American Chemical Society,**
**Washington D.C. 1973**

(73) Patentinhaber: **BAYER AG**
**Zentralbereich Patente, Marken und Lizenzen**
**D-5090 Leverkusen 1, Bayerwerk (DE)**

(72) Erfinder: **Buysch, Hans-Josef, Dr.**
**Brandenburger Strasse 28**
**D-4150 Krefeld 1 (DE)**
Erfinder: **Krimm, Heinrich, Dr.**
**Heyenbaumstrasse 65**
**D-4150 Krefeld 1 (DE)**
Erfinder: **Rudolph, Hans, Dr.**
**Haydnstrasse 9**
**D-4150 Krefeld 1 (DE)**

Courier Press, Leamington Spa, England.

Verfahren zur Abtrennung von Dimethylcarbonat aus seinem Azeotrop mit Methanol

Die Erfindung betrifft ein Verfahren zur Abtrennung von Dimethylcarbonat aus dem Dimethylcarbonat/Methanol-Azeotrop durch Destillation.

Dimethylcarbonat ist als Ausgangsstoff zur Herstellung aromatischer Carbonate, die ihrerseits wiederum Vorprodukte zur Herstellung von Polycarbonaten und Pflanzenschutzmitteln darstellen, von großer technischer Bedeutung (vgl. z.B. Ullmanns Encyklopädie der technischen Chemie, 3 Aufl., Bd. 9, S. 776 (1957), US—PS 3 933 846, DT—PS 971 790).

Dimethylcarbonat wird gemäß der US—PS 2 642 858, der US—PS 3 803 201 und der DT—OS 2 615 665 durch Umesterung cyclischer Carbonate, wie Äthylencarbonat, mit Alkoholen in Gegenwart von alkalischen Katalysatoren hergestellt. Hierbei wird ein etwa 30 Gew.-%iges Azeotrop mit Methanol erhalten.

Zur Gewinnung von Dimethylcarbonat aus dem Azeotrop wird gemäß der US—PS 3 803 201 durch Kühlen auf —70°C ein an Dimethylcarbonat reiches kristallines Produkt ausgefroren, aus dem Dimethylcarbonat durch Destillation gewonnen werden kann, Das Verfahren ist angesichts der erforderlichen tiefen Temperaturen und der technisch umständlichen Filtrationsstufe von geringem technischen Interesse.

Auch das Verfahren der DT—PS 2 450 856, nach dem eine Trennung durch eine Extraktivdestillation unter Verwendung von Wasser als Lösungsmittel möglich sein soll, ist in Anbetracht der hohen Wasserlöslichkeit und leichten Verseifbarkeit des Dimethylcarbonats verlustreich und somit unwirtschaftlich.

Ein weiteres Trennverfahren beruht auf einer Destillation bei einem Druck von etwa 10 atü, wobei nach Abdestillieren einer methanolreichen Kopffraktion Dimethylcarbonat als Rückstand erhalten wird (DT—OS 2 607 003).

Es wurde ein Verfahren zur Gewinnung von Dimethylcarbonat aus seinem Azeotrop mit Methanol durch Destillation gefunden, das dadurch gekennzeichnet ist, daß man das Azeotrop mit solchen Azeotropbildnern, die mit Methanol nicht mischbar sind, bei Normaldruck so destilliert dass ein niedrig siedendes Azeotrop aus dem betreffenden Azeotropbildner und Methanol bei einer Temperatur von 30 bis 63°C übergeht.

Durch die Mitverwendung von mit Methanol nicht mischbaren Azeotropbildnern bei der Destillation des Methanol/Dimethylcarbonat-Azeotrops destilliert zunächst ein niedrig siedendes Azeotrop aus dem betreffenden Gemisch des Azeotropbildners mit Methanol — der Siedepunkt des Azeotrops ist abhängig von der Art des verwendeten Azeotropbildners, er liegt im Temperaturbereich von 30°C bis 63°C — wodurch der Rückstand an Methanol verarmt. Zurück bleibt nahezu reines Dimethylcarbonat, das in den meisten Fällen keiner weiteren Reinigung durch Destillation mehr bedarf. Es war überraschend, daß diese Azeotropdestillation gelingt, obwohl bekanntermaßen die unten aufgeführten Azeotropbildner sowohl mit Methanol als auch mit dem Dimethylcarbonat azeotropsiedende Gemische bilden.

Das erfindungsgemäße Verfahren gewährleistet eine besonders wirtschaftliche, sauber und einfach durchzuführende Trennung von Methanol und Dimethylcarbonat.

Als Azeotropbildner seien zum Beispiel aliphatische Kohlenwasserstoffe mit 5—10, bevorzugt 6—7, Kohlenstoffatomen genannt, wie Pentan, Hexan, Heptan, Octan, 2-Methylhexan, 2-Methylheptan, 3-Methylheptan, 4-Methylheptan, 2,2-Dimethylpentan, 2,3-Dimethylpentan, 2,4-Dimethylpentan, 3,3-Dimethylpentan, 2,5-Dimethylhexan und 2,2,4-Trimethylpentan (Isooctan), ferner Kohlenwasserstoffe enthaltende technische Benzinfraktionen, wie Petroläther (Siedebereich 40 bis 80°C) Leichtbenzin (Siedebereich 60 bis 95°C) und Ligroin (Siedebereich 80 bis 110°C). Die Kohlenwasserstoffe und die Kohlenwasserstoffe enthaltenden technischen Benzinfraktionen können sowohl einzeln als auch im Gemisch untereinander als Azeotropbildner verwendet werden.

Bevorzug werden als Azeotropbildner eingesetzt: Hexan, Heptan, Octan, Isooctan, Petroläther, Leichtbenzin und/oder Ligroin, wobei Hexan und Heptan besonders bevorzugt sind.

Die Azeotropbildner werden zweckmäßig in solcher Menge eingesetzt, die eben ausreicht, um alles Methanol überzudestillieren. Die zuzusetzende Menge an Azeotropbildnern kann den bekannten Tabellen (vgl. z.B. Handbook of Chemistry and Physics, 51. Edition (1970), The Chemical Rubber Company, Cleveland/Ohio) mit den Angaben über die jeweilige Azeotropzusammensetzung entnommen oder durch einfache Vorversuche ermittelt werden.

Das zunächst über Kopf destillierende Azeotrop trennt sich in der Vorlage in eine im wesentlichen aus Methanol bestehende Phase, die wieder in die Dimethylcarbonat-Produktionsanlage zurückgeführt werden kann, und in eine im wesentlichen aus Kohlenwasserstoff(en) bestehende Phase, die in der Trennanlage wiederverwendet werden kann, auf.

Im Sumpf der Kolonne verbleibt als Rückstand Dimethylcarbonat, das gegebenenfalls durch Destillation gereinigt werden kann.

Das erfindungsgemäße Verfahren sei anhand der folgenden Beispiele verdeutlicht, ohne es jedoch auf diese Beispiele einzuschränken.

Beispiel 1
An einer mit Glasringen beschickten verspiegelten Füllkörperkolonne mit 1 m Füllhöhe wird ein aus 450 g Dimethylcarbonat/ Methanol-Azeotrop (30:70) und 297 g n-Heptan gestehendes Gemisch derart erhitzt, daß über Kopf ein im wesentlichen aus Methanol und Heptan bestehendes zweiphasiges Azeotrop bei 58,5°C abgetrennt wird. Sobald die Sumpftemperatur 83°C erreicht hat, wird die Kolonne entfernt und der Rückstand (86 g) über eine 10 cm hohe Kolonne fraktioniert. Bei 89—90°C gehen 63 g reines Dimethylcarbonat über. Der im Kolben verbleibende Rückstand (4,5 g) besteht ebenfalls aus reinem Dimethylcarbonat ($n_D^{20}$ 1,3688). Ausbeute 50% d.Th.

### Beispiel 2

Eine Mischung aus 150 g Dimethylcarbonat und 350 g Methanol wird mit 950 g n-Hexan an einer 40 cm hohen Silberspiegelkolonne derart destilliert, daß die Übergangstemperatur 50°C nich übersteigt. Der im Kolben verbleibende Rückstand (95 g) besteht nahezu aus reinem Dimethylcarbonat ($n_D^{20}$ 1,3675). Ausbeute 60% d.Th.

### Beispiel 3

Eine Mischung aus 45 g Dimethylcarbonat und 105 g Methanol wird mit 100 g Isooctan an einer 1 m hohen Silberspiegelkolonne derart fraktioniert, daß das zweiphasige Azeotrop bei 58,5°C übergeht. Die im Kolben zurückbleibende Flüssigkeit (20 g) ist nahezu reines Dimethylcarbonat ($n_D^{20}$ 1,3673). Ausbeute 40% d.Th.

### Beispiel 4

Ein aus 45 g Dimethylcarbonat und 105 g Methanol bestehendes Gemisch wird mit 85 g Ligroin vom Siedebereich 80—110°C über eine 50 cm hohe Silberspiegelkolonne im Laufe von 7 1/2 h destilliert, bis die Sumpftemperatur 90°C erreicht hat. Die im Kolben verbleibende Flüssigkeit (24 g) ist nahezu reines Dimethylcarbonat ($n_D^{20}$ 1,3676). Ausbeute 50% d.Th.

## Patentansprüche

1. Verfahren zur Gewinnung von Dimethylcarbonat aus seinem Azeotrop mit Methanol durch Destillation, dadurch gekennzeichnet, daß man das Methanol/Dimethylcarbonat-Azeotrop mit solchen Azeotropbildnern, die mit Methanol nicht mischbar sind, bei Normaldruck so destilliert, daß ein niedrigsiedendes Azeotrop aus dem betreffenden Azeotropbildner und Methanol bei einer Temperatur von 30 bis 63°C übergeht.

2. Verfahren nach Anspruch 1, dadurch gekennzeichnet, daß man als Azeotropbildner aliphatische Kohlenwasserstoffe mit 5 bis 10 Kohlenstoffatomen verwendet.

3. Verfahren nach Anspruch 1, dadurch gekennzeichnet, daß man als Azeotropbildner Kohlenwasserstoffe enthaltende technische Benzinfraktionen verwendet.

4. Verfahren nach Ansprüche 1 und 2, dadurch gekennzeichnet, daß man als aliphatische Kohlenwasserstoffe Hexan, Heptan, Octan und/oder Isooctan einsetzt.

5. Verfahren nach Ansprüche 1 und 3, dadurch gekennzeichnet, daß man als Kohlenwasserstoffe enthaltende technische Benzinfraktionen Petrolether, Leichtbenzin und/oder Ligroin einsetzt.

## Claims

1. Process for isolating dimethyl carbonate from its azeotrope with methanol by distillation, characterised in that the azeotrope is distilled under normal pressure with azeotropeforming agents which are methanol-immiscible.

2. Process according to Claim 1, characterised in that aliphatic hydrocarbons with 5—10 carbon atoms are used as the azeotrope-forming agents.

3. Process according to Claim 1, characterised in that industrial petrol fractions containing hydrocarbons are used as the azeotropeforming agents.

4. Process according to Claims 1 and 2, characterised in that hexane, heptane, octane and/or isooctane are employed as the aliphatic hydrocarbons.

5. Process according to Claims 1 and 3, characterised in that petroleum ether, light petrol and/or ligroin are employed as the industrial petrol fractions containing hydrocarbons.

## Revendications

1. Procédé pour isoler le carbonate de diméthyle de son azéotrope avec le méthanol par distillation, caractérisé en ce que l'on distille l'azéotrope à pression normale avec des composants formant des azéotropes et qui ne sont pas miscibles avec le méthanol.

2. Procédé selon la revendication 1, caractérisé en ce que l'on utilise en tant que composants formant des azéotropes des hydrocarbures aliphatiques en $C_5$—$C_{10}$.

3. Procédé selon la revendication 1, caractérisé en ce que l'on utilise en tant que composants formant des azéotropes des fractions industrielles d'essence contenant des hydrocarbures.

4. Procédé selon les revendications 1 et 2, caractérisé en ce que l'on utilise en tant qu'hydrocarbures aliphatiques l'hexane, l'heptane, l'octane et/ou l'iso-octane.

5. Procédé selon les revendications 1 et 3, caractérisé en que l'on utilise en tant que fractions industrielles d'essence contenant des hydrocarbures de l'éther de pétrole, de l'essence légère et/ou de l'essence moyenne.